# EUROPEAN PATENT SPECIFICATION

(11) **EP 2 633 846 B1**
(45) Date of publication and mention of the grant of the patent: **20.12.2017**
(21) Application number: 10858980.5
(22) Date of filing: 29.11.2010
(51) Int. Cl.: A61H 39/04

(54) **FINGER-PRESSURE TOOL FOR FACE**
FINGERDRUCKINSTRUMENT FÜR DAS GESICHT
OUTIL À PRESSION AU DOIGT POUR LE VISAGE

(30) Priority: 25.10.2010 JP 2010238437
(43) Date of publication of application: 04.09.2013
(73) Proprietor: Seilin & Co., Tokyo 153-0042 (JP)
(72) Inventor: TARUMI, Yoshiko, Tokyo 153-0042 (JP)
(74) Representative: Jacobacci Coralis Harle
(86) International application number: PCT/JP2010/071209
(87) International publication number: WO 2012/056594

(56) References cited:
- WO-A1-96/00108
- WO-A1-2005/051281
- JP-A- 9 299 441
- JP-A- H1 156 898
- JP-A- 11 076 290
- JP-U- H0 349 857
- JP-U- S63 137 649
- KR-B1- 100 954 673

## Description

The present invention relates to a facial finger-pressure tool, and more specifically to a facial finger-pressure tool which is capable of accurately applying pressing stimulation to a facial pressure point, and is effective, particularly, in reducing asthenopia.

It is commonly and widely known that a prolonged period of detailed-oriented manual work, personal computer work, automobile driving or TV viewing causes asthenopia and then headache, shoulder stiffness, etc.

Meanwhile there are several acupuncture points (pressure points) around a human eye, and stimulation of the acupuncture point (pressure point) improves particularly blood circulation around the eye, thereby making it possible to accelerate alleviation of asthenopia. Document WO2005051281 discloses a therapeutic beauty mask comprising a mask body, means for attaching and securing the body to cover at least a portion of a person's face, and a plurality of pressure points extending from an undersurface of the mask body, said pressure points being adapted or adjustable to coincide with known acupressure points of the human face. With a focus on this point, JP 08-304743A discloses an invention concerning an eyeglass designed to magnetize an inner surface of an eyeglass frame, i.e., a surface of an eyeglass frame facing a face of a user of the eyeglass.

Patent Document 1: JP 08-304743A

In the device disclosed in the above Patent Document, a magnetic source is disposed on an inner surface of a conventional eyeglass frame. However, as a way to improve blood circulation around an eye, it is also effective to apply finger-pressure to an acupuncture point (pressure point) around the eye, as well as the technique of applying a magnetic field thereto. A magnetic therapy needs to be continued for a certain period of time, because it does not have an immediate effect. The magnetic means is desirable as prophylactic use. However, in view of accelerating alleviation of asthenopia, it is most effective to directly stimulate an acupuncture point (pressure point) around an eye.

The conventional device is not provided with any member for stimulating the acupuncture point (pressure point), i.e., adapted to be brought into direct and press contact with a face. Thus, there is a problem that such a finger-pressure effect cannot be expected.

Moreover, the conventional device is intended to offer improvement based on an eyeglass frame, so that it is an uninteresting article to non-eyeglass wearers.

The present invention has been made with a focus on the above problem of the conventional technique, and proposed with a view to providing a facial finger-pressure tool capable of accelerating the alleviation of asthenopia.

In order to achieve the above object, the present invention provides a facial finger-pressure tool according to claim 1.

The present invention provides a facial finger-pressure tool which comprises: a goggles frame; and a finger-pressure protrusion provided on an inner surface of a front portion and a temple portion of the goggles frame, at a position corresponding to at least one acupuncture point around an eye, wherein the goggles frame is made of a flexible material, and the temple portion has two free ends provided with tightening means adapted to forcibly bring the goggles frame into close contact with face and head regions.

The finger-pressure protrusion is attached adjustably and movably in conformity to a position of the acupuncture point.

The finger-pressure protrusion is detachably attached.

The goggles frame is made of a flexible material which is at least one selected from the group consisting of polyvinyl chloride, soft synthetic resin, natural rubber, and synthetic rubber including chloroprene rubber, silicon rubber, acrylic rubber and urethane rubber.

The finger-pressure protrusion is comprised of a metal tablet or a magnet.

The finger-pressure protrusion is provided on the inner surface of the front portion and the temple portion of the goggles frame, at a position corresponding to at least one acupuncture point around an eye, and the goggles frame is made of a flexible material, so that the finger-pressure protrusion can be pressed against the acupuncture point to apply adequate stimulation to the acupuncture point, which is significantly effective in curing asthenopia. In addition, when a user feels that the stimulation is insufficient in a normal usage state, he/she can press the goggles frame from an outside thereof toward the face by the palm. This provides stronger stimulation and an enhanced finger-pressure effect.

The tightening means is provided at the two free ends of the temple portion, so that a level of tightness can be adjusted to adjust a level of press contact (force) of the finger-pressure protrusion against the facial acupuncture point. That is, when the tightening means is tightly fastened (tied), the goggles frame made of a flexible material is more strongly brought into close contact with face and head regions to increase the press contact force of the finger-pressure protrusion against the facial acupuncture point, thereby providing a further enhanced stimulation (finger-pressure) effect. Further, the adjustment of the tightness makes it possible to obtain any level of finger-pressure effect according to user's preference.

The facial finger-pressure tool consists of a front frame which is adapted to cover an eye region and provided with a finger-pressure protrusion on an inner surface of the front portion, at a position corresponding to at least one acupuncture point around an eye, wherein the front frame is made of a flexible material.

Thus, for example, a cord or a tensile member such as a rubber band may be attached to the front frame to allow the front frame to be brought into press contact with the face. Alternatively, the front frame may be used in a state in which it is interposed between the face and existing swimming goggles, 3D TV glasses or the like. Further, it may be used in various other manners. For example, a user may place only this facial finger-pressure tool on the eye region, and then move the palm in a direction for pressing the front portion and in a direction away from the front portion.

The facial finger-pressure tool in an embodiment, which is not part of the present invention, can be formed as an eye mask-like, i.e., wide-width body. Thus, a large number of finger-pressure protrusions may be provided thereon. In this case, the finger-pressure protrusions can be brought into contact with points other than acupuncture points, so that beauty and health effects of facial massage can be expected in addition to stimulation of acupuncture points.

The finger-pressure protrusion is attached in a positionally changeable manner. Thus, the position of the finger-pressure protrusion can be finely adjusted to adequately conform to a position of the acupuncture point. This makes it possible to obtain a further adequate finger-pressure effect.

The finger-pressure protrusion is detachably attached. Thus, a size, such as contact area and/or height dimension, of the finger-pressure protrusion can be freely changed to obtain an adequate finger-pressure effect.

The front portion and the temple portion of the goggles frame are made of a flexible material which is at least one selected from the group consisting of polyvinyl chloride, soft synthetic resin, natural rubber, and synthetic rubber including chloroprene rubber, silicon rubber, acrylic rubber and urethane rubber, so that the goggles frame can be brought into close contact with the face to obtain an adequate finger-pressure effect.

The finger-pressure protrusion is comprised of a magnet, or "noirokorun (trade name) " which is a metal tablet, so that it becomes possible to apply stimulation to an acupuncture point in a close contact state with the face, thereby obtaining an adequate finger-pressure effect. This metal tablet is a metal tablet-shaped body, and used in physical therapy as an applied technique of principles of acupuncture and finger-pressure.

The invention will now be described in relation to the following Figures:
FIG. 1 is a perspective view illustrating a first embodiment of the present invention.
FIG. 2 is an explanatory diagram illustrating an example of attachment of a finger-pressure protrusion.
FIG. 3 is a perspective view illustrating an embodiment, which is not part of the present invention, but can be useful for understanding the invention.
FIG. 4 is a perspective view of an embodiment, which is not part of the present invention, but can be useful for understanding the invention.

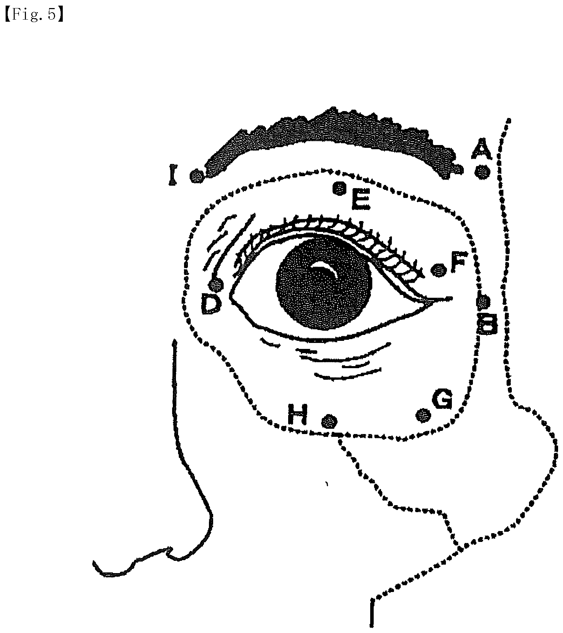

FIG. 5 is a diagram illustrating acupuncture points around an eye.

An embodiment of the present invention will now be described based on the drawings.

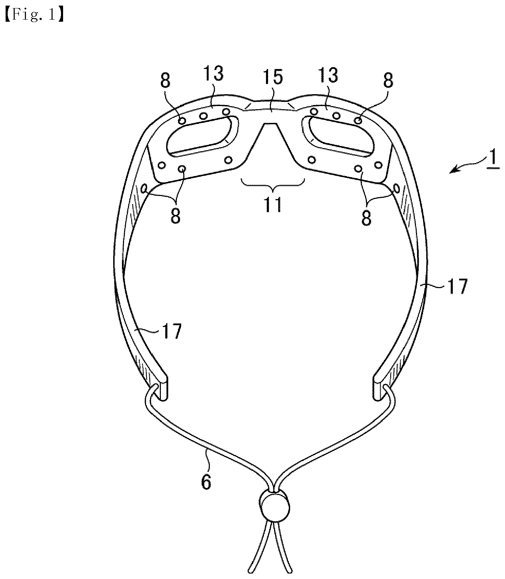

FIG. 1 illustrates a first embodiment of the present invention, wherein a goggles frame 1 as a face finger-pressure tool comprises a front portion 11 and a pair of temple portions 17. The front portion 11 is comprised of a pair of rims 13 and a bridge 15 connecting the rims 13 together, as called in the field of eyeglass frames. In this embodiment, no nose pad is provided. Thus, an inner (face-facing) surface of the front portion 11 is brought into close contact with a face, as described later.

The temple portions 17 are integrally connected, respectively, to opposite ends of the front portion 11. The front portion 11 and the pair of the temple portions 17 may be integrally molded in a single piece, or may be molded separately and subsequently connected together. In the case where they are molded separately, it is desirable to connect them together by means of hinge connection or the like.

A pair of tightening members 6 such as rubber belts or straps are attached, respectively, to ear-side ends, i.e., free ends, of the temple portions 17. When the right and left tightening members 6 are integrally fastened (locked) on a rear side of a head, the goggles frame is brought into close contact with face and temporal regions. As the tightening members are tightly fastened (tied), a level of press contact of an aftermentioned finger-pressure protrusion 8 becomes higher, and a level of stimulation becomes stronger in proportion thereto. Thus, a user can adjustably increase and reduce a level of the tightness according to his/her preference.

The front portion 11 and the temple portions 17 of the goggles frame are made of a flexible material which is at least one selected from the group consisting of: soft synthetic resin such as polyurethane, polyvinyl chloride or acrylonitrile; and natural or synthetic rubber such as silicon rubber or chloroprene rubber.

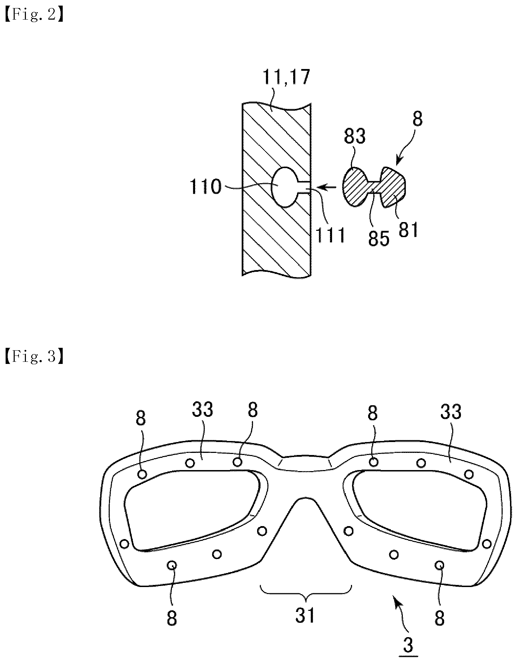

A protrusion 8 for a finger-pressure effect (finger-pressure protrusion 8) is attached to the inner surface of the front portion 11 and the temple portions 17. The attachment position of the finger-pressure protrusion 8 corresponds to: an acupuncture point (pressure point), such as "seimei" (a depressed region located slightly away from inner corners of right and left eyes toward a nose), "gyoyou" (a position just below a center of an eyebrow),"taiyou" (a position close to a tail of the eye with respect to a temple), or "shihaku" (a position just below a center of a pupil); or an acupuncture point (pressure point) around the eye, such as "shichikuku", "doushiryou", "joumei", "gaimei", "kyugo", "shoukyu" or "sanchiku" (see FIG.2). Asthenopia, and headache, shoulder stiffness, etc., to be caused by the asthenopia, can be alleviated by applying stimulation to such an acupuncture point.

FIG. 2 illustrates one example of attachment of the finger-pressure protrusion 8, wherein the finger-pressure protrusion 8 comprises a pressing portion 81, a fitting engagement portion 83, and a bridge portion 85 connecting them together. A hole 110 for holding the finger-pressure protrusion 8 is formed in the inner surface of the front portion 11 and the temple portion 17, at a position corresponding to the acupuncture point. The hole 110 is configured to have a size capable of fastenably receiving therein the fitting engagement portion 83 of the finger-pressure protrusion 8.

An inlet portion 111 of the hole 110 is formed to have a reduced diameter, thereby fastenably holding the bridge portion 85 of the finger-pressure protrusion 8. This prevents the finger-pressure protrusion 8 from being easily unfastened or disengaged from the inner surface of the front portion 11 and the temple portion 17. Alternatively, the finger-pressure protrusion 8 may be adhesively attached, for example, using an adhesive seal.

A plurality of types of the finger-pressure protrusions 8 each different in size, such as contact area and/or height dimension, of the pressing portion 81 to be brought into contact with the facial acupuncture point, may be prepared in advance. In this case, a level of finger-pressure can be freely adjusted according to user's preference.

Further, the hole 110 (111) formed in the front portion 11 and the temple portions 17 may be formed in a shape extending in a right-left or front-rear direction and/or an up-down direction, e.g., a cross shape. In this case, the finger-pressure protrusion 8 can be moved in the right-left or front-rear direction and/or the up-down direction along the groove, which makes it possible to adequately conform to a position of the acupuncture point in an easy manner.

The finger-pressure protrusion 8 should be provided in a number equal to that of the acupuncture points as a minimum requirement. However, the present invention is not limited thereto, but the finger-pressure protrusion 8 may be provided at a position other than the positions of the acupuncture points. In this case, beauty and health effects as effects of massaging the entire eye region can be expected.

The finger-pressure protrusion 8 desirably made of, but is not particularly limited to, a metal material, preferably, a metal tablet "noirokorun (trade name)" which is a magnetized metal material. That is, in cases where it is desired to obtain only a finger-pressure effect without any need for magnetic therapy, a substitute material such as a hard plastic may be used.

The above tightening members 6 are not provided in the facial finger-pressure tool 1 in claim 1. In this case, a user may press the front portion 11 and/or the temple portions 17 by his/her palms, to obtain a finger-pressure effect. As an example of the tightening member, it is possible to use a rubber band, a strap or a cord.

While any object, such as a lens, is not installed inside each of the rims 13 of the front portion 11 of the facial finger-pressure tool, a lens for visual correction, a lens for 3D glasses or the like may be installed. In this case, it is necessary to additionally take measure to provide conventional lens fixing/holding means to the rims.

FIG. 3 illustrates an embodiment, which is not part of the present invention, but can be useful for understanding the invention, wherein a facial finger-pressure tool 3 consists only of a front frame 31. The front frame 31 is comprised of a pair of rims 33 and a bridge 35 integrally connecting the rims 33 together. That is, the facial finger-pressure tool is similar to horn-rimmed glasses in terms of configuration.

As with the first embodiment, a finger-pressure protrusion 8 is installed to an inner surface of the front frame 31. As with the first embodiment, the finger-pressure protrusion 8 may be fastenably attached, or may be detachably attached, or may be configured to be displaceable along the inner surface in conformity to a position of the acupuncture point. The front frame 31 may have a hole provided at each of opposite ends thereof to allow a rubber belt, a strap or the like to be attached thereto.

In the facial finger-pressure tool illustrated in FIG. 3, the front frame 31 can be fixed to a face by a rubber belt, a strap or the like. In the case where such fixing means is not used, the front frame 31 may be interposed between a face and 3D TV glasses, swimming goggles or the like, in such a manner as to be brought into press contact with the face by means of a holding force of the 3D TV glasses, the swimming goggles or the like, to obtain a finger-pressure effect. For this purpose, the front frame 31 is in a plain state in which no lens is installed.

In the case of using swimming goggles, it is necessary to take measures to allow the facial finger-pressure tool 3 to be reliably received in the swimming goggles, while preventing intrusion of water through the swimming goggles. Such water-tightness can be ensured by forming them using substantially the same material.

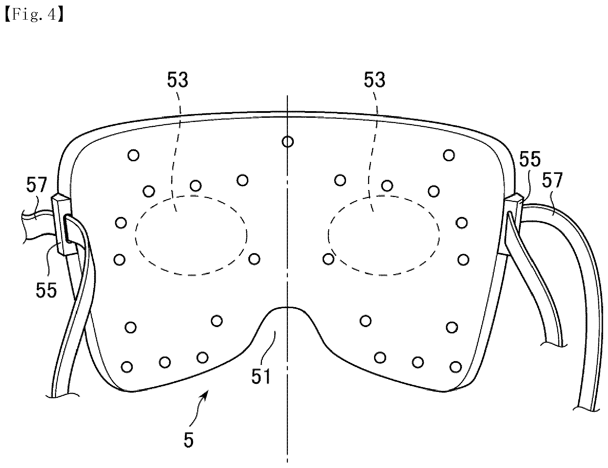

FIG. 4 illustrates an embodiment, which is not part of the present invention, but can be useful for understanding the invention, wherein the facial finger-pressure tool 5 is formed as an eye mask-like body adapted to cover a part of a cheek region as well as an eye region. In the illustrated embodiment, the reference numeral 51 indicates a region for allowing a nose to be exposed. The reference numeral 53 indicates a transparent portion for allowing an eye to be exposed. Alternatively, the transparent portion 53 may be omitted as in a sleeping eye mask.

The reference numeral 55 indicates each of a pair of strap mounts, and the reference numeral 57 indicates each of a pair of straps to be fastened together on a rear side of a head so as to maintain the facial finger-pressure tool 5 in a contact state with the face. In the case where the facial finger-pressure tool 5 is not held by the head, the straps 57 may be omitted, and the facial finger-pressure tool 5 may be used for massage in such a manner that it is placed on a face and pressed by a palm. It is to be understood that, even in the case where the facial finger-pressure tool 5 is kept in close contact with the face by the straps, it may be pressed by a palm when it is desired to apply a stronger pressing force.

A finger-pressure protrusion 8 is provided on an inner surface of the facial finger-pressure tool 5. The finger-pressure protrusion 8 is provided at a position corresponding to that of the acupuncture point as with the above embodiments. Differently from the facial finger-pressure tool illustrated in FIG. 1 or 3, the facial finger-pressure tool 5 has a wider contact area with a face. Thus, the finger-pressure protrusion 8 is appropriately provided at a position other than that of the acupuncture point. That is, a finger-pressure protrusion at a position corresponding to the acupuncture point is used for stimulation of a pressure point, and a finger-pressure protrusion at other position is used for a facial massage.

As with the above embodiments, the facial finger-pressure tool 5 is made of at least one selected from the group consisting of polyvinyl chloride, soft synthetic resin, natural rubber, and synthetic rubber such as chloroprene rubber, silicon rubber, acrylic rubber and urethane rubber. As means for attachment of the finger-pressure protrusion 8, the configuration as illustrated in FIG. 2, or adhesive attachment, may be employed. Further, the finger-pressure protrusion 8 may be comprised of a magnet.

This embodiment is not limited to the eye mask-like body, but may be a Noh mask-like body adapted to cover the entire face. In this case, a massage effect for the entire face can be obtained, which is advantageous to beauty and health. The massage may be performed in such a manner that the facial finger-pressure tool is pressed by a palm, or lightly touched by a vibrator type electric massaging device.

To help understanding of the invention, explanations of codes are now given:
1: facial finger-pressure tool
11: front portion
17: temple portion
3: facial finger-pressure tool
31: front frame
33: rim
35: bridge
5: facial finger-pressure tool
53: transparent portion
55: strap mount
57: strap
6: tightening member
8: finger-pressure protrusion
81: pressing portion
83: fitting engagement portion
85: bridge portion
A: shichikuku
B: doushiryou
D: seimei
E: joumei
F: gaimei
G: kyugo
H: shoukyu
I: sanchiku

## Claims

1. A facial finger-pressure tool comprising: a goggles frame (1) made of a flexible material having a front portion (11) and two temple portions (17); and at least one finger-pressure protrusion (8) provided on an inner surface of the front portion at a position corresponding to at least one acupuncture point around an eye, wherein said front portion of the goggles frame has holes (110) which are formed in the inner surface of the front portion at the positions corresponding to the acupuncture points or at other positions, and the finger-pressure protrusion (8) is attached in conformity to a position of any one of the holes, the facial finger-pressure tool being **characterized in that** each of said temple portions of the goggles frame has a hole which is formed in the inner surface of the temple portions at the position corresponding to the acupuncture point or at other positions, each of the holes having an inlet portion (111) reduced in diameter so as to fastenably receive therein a fitting engagement portion (83) of the finger-pressure protrusion, and the finger-pressure protrusions (8) are attached removably to the holes of the temple portions, respectively, and that the facial finger-pressure tool comprises a tightening means having a pair of tightening members (6) attached, respectively, to free ends of the temple portions, said tightening means adapted to forcibly bring the goggles frame into close contact with face and head regions so that a level of press contact of the finger-pressure protrusion is adjustable by adjustably increasing and reducing a level of the fastening tightness between the pair of the tightening members according to user's preference.

2. The facial finger-pressure tool as defined in claim 1, wherein a shape of the goggles frame is formed in a shape of an eye mask adapted to cover an eye region.

3. The facial finger-pressure tool as defined in any one of claims 1 and 2, wherein the finger-pressure protrusion is made of a material which is one selected from the group consisting of metal, hard plastic, magnetized metal, noirokorun, which is a metal tablet.

## Patentansprüche

1. Fingerdruckinstrument für das Gesicht, das ein Brillengestell (1) aus einem flexiblen Material mit einem Stirnteil (11) und zwei Bügelteilen (17), sowie wenigstens eine an einer inneren Oberfläche des Stirnteils in einer wenigstens einem Akupunkturpunkt um ein Auge entsprechenden Position vorgesehene Fingerdruckerhebung (8) aufweist, wobei der Stirnteil des Brillengestells Löcher (110) aufweist, die in der inneren Oberfläche des Stirnteils an den Positionen, die den Akupunkturpunkten entsprechen, oder an anderen Positionen gebildet sind, und wobei die Fingerdruckerhebung (8) in entsprechender Weise an der Position irgendeines der Löcher angebracht ist, wobei das Fingerdruckinstrument für das Gesicht **dadurch gekennzeichnet ist, daß** jeder der Bügelteile des Brillengestells ein Loch aufweist, das in der inneren Oberfläche der Bügelteile an der Position, die dem Akupunkturpunkt entspricht, oder an anderen Positionen gebildet ist, wobei jedes der Löcher einen Eingangsabschnitt (111) aufweist, dessen Durchmesser so reduziert ist, daß darin ein passender Montageabschnitt (83) der Fingerdruckerhebung montierbar aufgenommen werden kann, wobei die Fingerdruckerhebungen (8) jeweils abnehmbar in den Löchern der Bügelteile angebracht sind, und daß das Fingerdruckinstrument für das Gesicht Spannmittel mit einem Paar jeweils an freien Enden der Bügelteile befestigten Spannelementen (6) aufweist, wobei die Spannmittel dazu ausgelegt sind, das Brillengestell in engen Kontakt mit Bereichen des Gesichts und des Kopfes zu bringen, so daß ein Grad des Druckkontakts der Fingerdruckerhebung durch ein einstellbares Erhöhen oder Senken eines Maßes an Befestigungsspannung zwischen dem Paar Spannelementen gemäß dem Vorzug des Benutzers einstellbar ist.

2. Fingerdruckinstrument für das Gesicht gemäß Anspruch 1, **dadurch gekennzeichnet, daß** eine Form des Brillengestells in Form einer Augenmaske ausgebildet ist, die dazu ausgelegt ist, einen Augenbereich abzudecken.

3. Fingerdruckinstrument für das Gesicht gemäß einem der Ansprüche 1 und 2, **dadurch gekennzeichnet, daß** die Fingerdruckerhebung aus einem Material ist, das aus der aus Metall, Hartplastikmaterial, magnetisiertem Material und "Noirokorun", das ein Metallplättchen ist, bestehenden Gruppe ausgewählt ist.

## Revendications

1. Outil de digitopuncture faciale comprenant : une structure de type lunettes (1) constituée d'un matériau souple ayant une partie avant (1) et deux parties formant branches (17) ; et au moins une protubérance de digitopuncture (8) prévue sur une surface interne de la partie avant, à une position correspondant à au moins un point d'acupuncture autour d'un oeil, ladite partie avant de la structure de type lunettes ayant des trous (110) formés dans la surface interne de la partie avant, aux positions correspondant aux points d'acupuncture ou à d'autres positions, et la protubérance de digitopuncture (8) étant fixée conformément à une position de l'un quelconque des trous, l'outil de digitopuncture faciale étant **caractérisé en ce que** chacune desdites parties formant branches de la structure de type lunettes a un trou formé dans la surface interne des parties formant branches, à la position correspondant au point d'acupuncture ou à d'autres positions, chacun des trous ayant une partie d'entrée (111) de diamètre réduit de façon à recevoir fixement une partie d'emboîtement (83) de la protubérance de digitopuncture, et les protubérances de digitopuncture (8) sont attachées de manière amovible aux trous respectifs des parties formant branches, et **en ce que** l'outil de digitopuncture faciale comprend des moyens de serrage ayant une paire d'éléments de serrage (6) respectivement attachés aux extrémités libres des parties formant branches, lesdits moyens de serrage étant adaptés pour forcer la structure de type lunettes à entrer en contact étroit avec des régions du visage et de la tête de telle sorte qu'un niveau de contact par pression de la protubérance de digitopuncture puisse être ajusté par augmentation ou diminution ajustable d'un niveau de serrage de fixation entre les deux éléments de serrage de la paire selon les préférences de l'utilisateur.

2. Outil de digitopuncture faciale selon la revendication 1, dans lequel la structure de type lunettes est façonnée sous la forme d'un masque pour les yeux adapté pour recouvrir une région oculaire.

3. Outil de digitopuncture faciale selon l'une quelconque des revendications 1 et 2, dans lequel la protubérance de digitopuncture est constituée d'un matériau sélectionné dans le groupe comprenant le métal, le plastique dur, le métal magnétisé, le noirokorun, qui est une pastille métallique.
